# EUROPEAN PATENT SPECIFICATION

(11) **EP 0 814 719 B1**
(45) Date of publication and mention of the grant of the patent: **08.10.2003**
(21) Application number: 96908664.4
(22) Date of filing: 06.03.1996
(51) Int. Cl.: A61B 19/00, A61N 7/00

(54) **ULTRASOUND THERAPY DEVICE**
ULTRASCHALLTHERAPIEVORRICHTUNG
APPAREIL A ULTRASONS A USAGE THERAPEUTIQUE

(30) Priority: 08.03.1995 JP 4871095; 05.03.1996 US 611105
(43) Date of publication of application: 07.01.1998
(73) Proprietor: Ekos Corporation, Bothell, WA 98021 (US)
(72) Inventor: TACHIBANA, Katsuro, Fukuoka-shi, Fukuoka 810 (JP); TACHIBANA, Shunro, Fukuoka-shi, Fukuoka 810 (JP)
(74) Representative: Horner, David Richard
(86) International application number: US9603010
(87) International publication number: WO96027341

(56) References cited:
- EP-A- 0 668 052
- WO-A-92/07622
- WO-A-93/08738
- DE-A- 3 919 592
- US-A- 4 582 067
- US-A- 4 971 991
- US-A- 5 149 319
- US-A- 5 158 071

## Description

### BACKGROUND OF THE INVENTION

This ultrasound therapy device can be used for thrombosis, atherosclerosis and other diseases and for the purpose of therapy of re-occlusion after angioplasty treatment with the PTCA, DCA, stent, laser, rotors, etc. The invention can be especially used by a catheter with an ultrasound emitting element mounted at the tip which can expose ultrasound to the light sensitive drug.

Previous technology: For example, in the case of acute myocardial infarction, the coronary artery can be clogged by thrombus or atherosclerotic changes, thus preventing oxygen supply to the heart muscle resulting in necrosis.

One method of treatment for myocardial infarction in internal medicine is administration of thrombolytic agents. This means of therapy is to administrate drugs orally or by injection to dissolve the clots. However, this non-surgical thrombus dissolving treatment drug circulates within the whole body, preventing adequate drug absorption to the target. Thus, the amount of drug dosage at the lesion is very small compared to total administration volume and is considered a problem as an efficient drug administration method. Also, increasing the drug concentration to obtain effects may result in severe side effects in other organs.

On the other hand, such other methods as mechanical blood dilation method, are known to locally treat thrombo-atherosclerotic lesions. Blood vessel dilatation treatment, called PTCA (percutaneous transluminal coronary angioplasty) is done under x-ray by inserting a guide wire percutaneously from the femoral artery to the coronary artery to deliver a catheter with a balloon at the tip which can be inflated. This balloon can dilate the stenotic lesion resulting in an increase of blood flow. There are other methods such as laser and drills to ablate the lesion.

However, after these procedures, restenosis occurs in several months after the treatment in as high a rate as 30%-50% of the patients. A routine check up after treatment by coronary angiography is repeated, if there is restenosis, angioplasty is repeated. Some cases need another dilatation treatment and in cases where there is impossible, coronary bypass surgery is performed.

In order to prevent restenosis, there is a method of placing at the location of stenosis, a metal net like cylinder device called stents, which supports vascular wall after dilatation by a balloon catheter. Still, the rate of occurrence of restenosis is high.

Restenosis is induced by injury of the inner wall of the blood vessel made while inserting the balloon or stents. It is thought that neointimal hyperplasia is the cause of restenosis.

Recently, in order to prevent restenosis, photodynamic therapy using light sensitive drugs is under investigation with such drugs as Photofrin, UpD, DHE/E (dihematoporphrin and ether), Chloroalunum sulfonated phthalocyanine, 8-methoxypsoralen, 5-amino-levulinic acid, etc.. See Paolo Ortu, et al., Photodynamic Therapy of Arteries, Circulation, American Heart Association Journal (1992, vol. 85, pp. 1189-1196).

Light sensitive drugs are activated when exposed to light and becomes drug effective. Thus, after administration of the drug into the body, light exposure to the target lesion will induce drug effectiveness only at that location. Thus. there is the benefit that photodynamic therapy using light sensitive drugs are less apt to have side effects to non-diseased portions.

However, there is a problem in that there is a need to expose light from outside the body in the case of photodynamic therapy. As light only penetrates at the most 1 cm into human tissues, therapeutic effects can only be obtained at locations near the surface of the body. In addition. when we try to penetrate light to a greater distance from the surface of the body, there is a need to increase the exposure energy. In this case, there was a possibility that the patient will experience heat and degeneration of tissues. There is the problem of higher cost due to enlargement of the device. In addition, there is the problem of light absorption by the red blood cells when photodynamic therapy is used as a blood vessel treatment In current photodynamic therapy of blood vessels, blood stream is stopped at the lesion and a transparent liquid is replaced which is a complicated procedure.

Document WO-A-93/08738 discloses a catheter having an ultrasound emitter at the tip of the catheter and a longitudinally extending lumen.

### SUMMARY OF THE INVENTION

The object of the tool of the invention as defined in claim 1 is to provide a drug delivery tool to locally treat diseases without the use of light energy. Preferred embodiments of this tool are defined in the dependent claims.

The ultrasound catheter can be inserted into the blood vessel to expose ultrasound to light sensitive drugs and activate the light sensitive drug.

The light sensitive drug can be delivered via the catheter or by conventional injection to the blood stream. The ultrasound emitter at the tip of the catheter can activate the light sensitive drug inside the blood vessel. The drug can be activated by ultrasound energy as well as light energy so the light sensitive drugs are activated by ultrasound. Thus, light sensitive drugs only become effective near the diseased location and makes it possible to treat site specifically.

Additionally, it is possible for ultrasound energy to be exposed from outside the body. Compared to light energy, ultrasound can reach further and deeper located lesions from the surface of the body because there is less energy decrease while penetrating the tissue, making is possible to activate light sensitive drugs at the lesion.

### BRIEF DESCRIPTION OF THE FIGURES

Fig. 1 is a schematic of the ultrasound therapy device of the present invention.
Fig. 2 is an enlarged diagram of the cross-section of the ultrasound generating device.
Fig. 3 is a schematic of the hyperplasia of blood vessels.
Figs. 4a-b are cross-views of the Fig. 3 blood vessel XX line and YY line cut at right angle of the longitude axis of the vessel.

### DETAILED DESCRIPTION OF THE PREFERRED EMBODIMENT

Example of Use: The following is a specific and detailed description with diagrams of an example of the use of the invention.

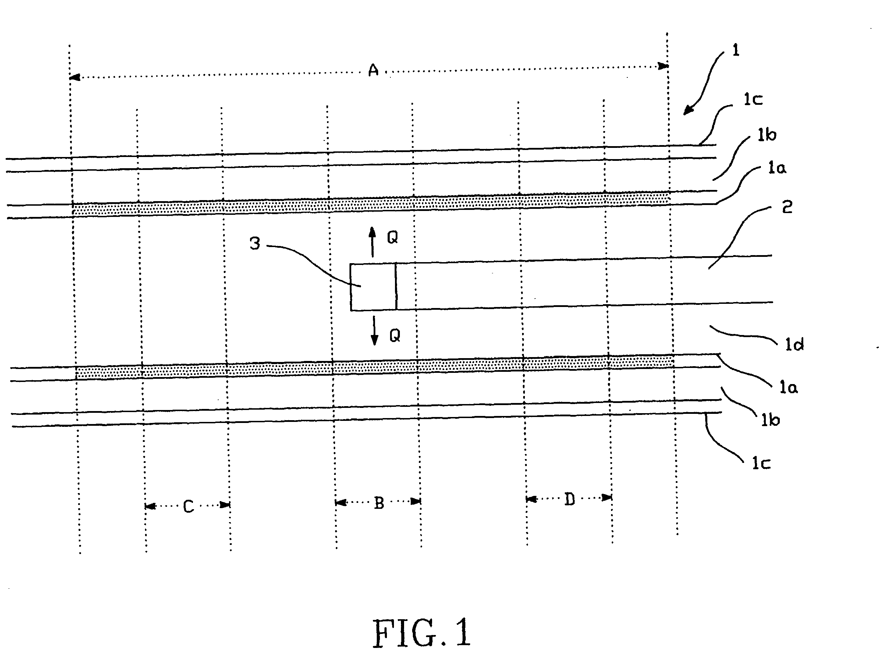

Figure 1 shows a schematic of how the ultrasound device of the invention can be used. The catheter device 2 made from Teflon or silicone has an ultrasound emitting element 3 at the tip, which is inserted into the blood vessel 1.

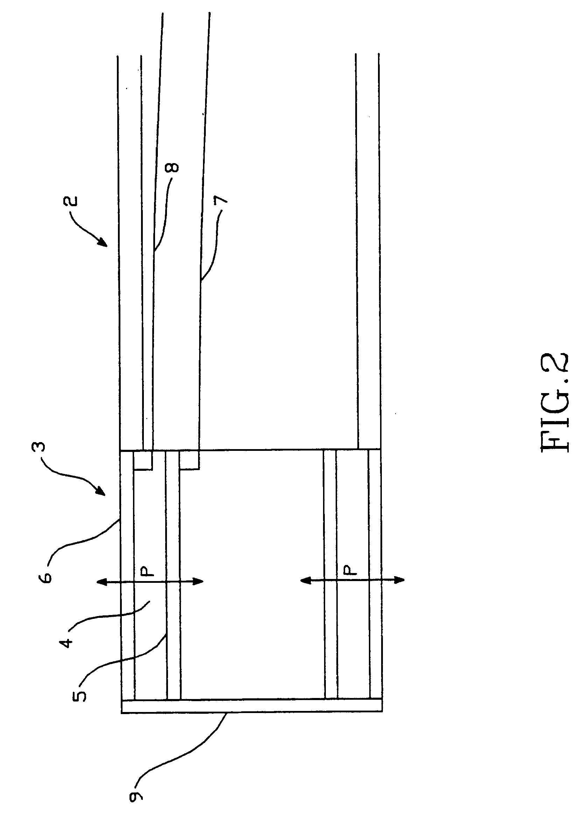

Figure 2 shows an enlarged cross-sectional view of the device 3. The ultrasound emitting element 3, a cylinder like electrical element 4, the inner side of the element surface 4 has for instance a silver electrode 5 shaped as a cylinder. The outer surface element 4 is similarly plastered in a cylinder like shape as an outer electrode 6. in addition, the inner 5 and outer 6 surface of the electrodes are coated as to maintain electrical isolation from the outside. Both the electrodes are connected to a lead wire 7 and 8. The lead wires 7 and 8 both go along through the inside of the catheter 2 to the root of the catheter outside the body. Also, the tip of the ultrasound generating device is capsulated in a circular disk 9. The diameter of the tip of the catheter can be about 1mm and 1 mm in length in the longitude axis direction. However, the size can be varied differently according to the type or state of the disease lesion and is not limited to this specific size.

Electrical signals at ultrasound frequencies is inputted between electrodes 5 and 6 of the ultrasound generating device 3 to permit the electrical element 4 to generate ultrasound in the direction of arrow P by physical vibration. The ultrasound vibration will be exposed to the light sensitive drug in the blood stream 1d within the blood vessel I and in the intimal layer 1a of the blood vessel wall. Ultrasound will be transmitted in the direction Q as shown in Figure 1 at a right angle of the ultrasound element surface 4. In other words, ultrasound will be radiated at a right angle of the axis of the ultrasound generating device 3 thus efficiently exposing ultrasound vibration to the intimal layer. When the intimal layer 1a is exposed to ultrasound vibration, tissues relax by ultrasound making drugs to be absorbed easier.

### Experiment Example:

The following is an experiment which proves that there was inhibiting effects of neointimal hyperplasia using this ultrasound generating device described above (experiment A).

The abdominal aorta intimal layer 1a of Japanese white rabbits were balloon injured (Fogarty, 4f) for a length of 7 cm under anesthesia by Nembutal (24mg/kg). Figure 1 shows the range of intimal layer injury of the blood vessel. lb is the smooth muscles layer, and Ic is the external layer. Immediately after injury of the artery, Photofrin II (5mg/kg) known as a light sensitive drug, was injected intravenously from the ear. At the same time, as shown in diagram 1, the ultrasound generating device was used to expose ultrasound for 10 minutes at the center 1 cm length of the injured artery. B in diagram 1 was ultrasound exposed and 1d contains light sensitive drugs in the blood. Ultrasound intensity was 0.3W/cm² and frequency 1.3 MHz.

Four weeks after, the ultrasound exposed portion B and the non-ultrasound portion B and C were incised, cross-cut and stained with EVG stain. The hyperplasia area was measured by a computer (Macintosh Quadra 800) for graphical analysis. The ratio of intimal area and media area was measured.

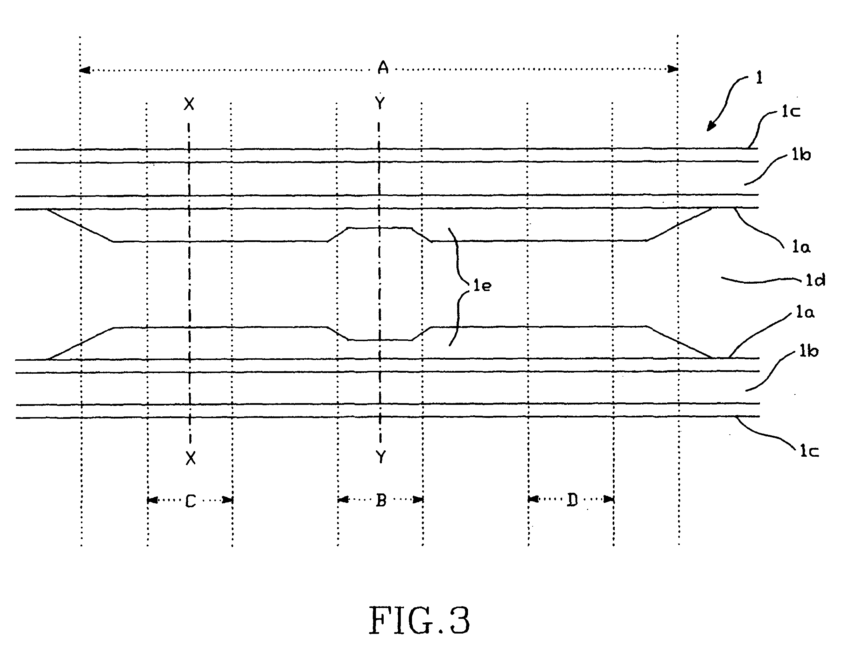

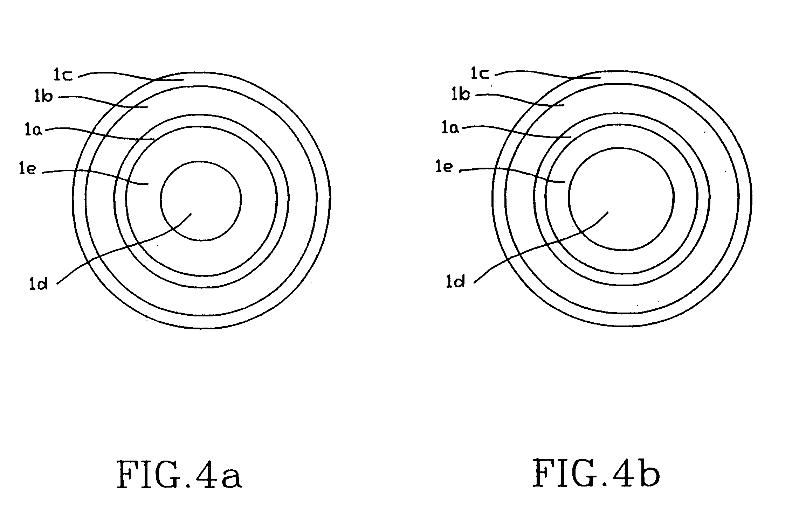

Figure 3 shows a scheme of the state of hyperplasia. Figure 4(a), (b), shows cross-sectional views cut in the right angle of the longitude direction as X-X line and Y-Y line.

Additionally, in order to determine the effects of ultrasound alone, immediately after blood vessel wall injury the same procedure was performed, however, without administration of Photofrin II (experiment B).

Comparison were performed with student's t test (paired, 0<0.05) between ultrasound and non-ultrasound exposed samples. ANOVA was used for other comparisons.

The neointimal area was defined as the cross-sectional area of the surface to the intima layer and medial area between the intima to the external layer.

The results are shown below:

**Table 1**

| | | Intimal/Medial Area Ratio | Difference |
|---|---|---|---|
| Experiment A N=9 | Ultrasound+Photofrin II | 0.24 ± 0.12 | positive |
| | Photofrin II alone | 0.55 ± 0.12 | |
| Experiment B N=7 | Ultrasound alone | 0.53 ± 0.21 | negative |
| | Non-treated | 0.59 ± 0.19 | |

Results from experiments A and B show that Photofrin II alone or ultrasound alone had no suppressing effect of hyperplasia. However, ultrasound exposure and Photofrin II administration resulted in significant therapeutic effects.

In the above case, ultrasound was exposed to the blood vessel from outside the body, however as described in detail in U.S. Patent No. 5,158.071, ultrasound can be focused to a certain location inside the body by extracorporeal ultrasound energy irradiation to light sensitive drugs.

### The Effects of the Invention

As described above, as light sensitive drugs are also sensitive to ultrasound, using the tool of the invention ultrasound is applied instead of light. Diseased lesions far at a distance from the surface of the body can be treated locally with the effect of drugs effectively for treatment.

The foregoing description of a preferred embodiment of the invention has been presented for purposes of illustration and description. It is not intended to be exhaustive or to limit the invention to the precise forms disclosed. Obviously, many modifications and variations will be apparent to practitioners skilled in this art. The scope of the invention is defined by the following claims.

## Claims

1. A drug delivery tool, comprising:
a drug delivery cathete (2) for insertion into a patient's body, the drug delivery catheter (2) having a distal end;
a light sensitive drug that is activatable by ultrasound energy, the light sensitive drug being introducible into the patient's body through the drug delivery catheter (2), wherein at least a portion of the light sensitive drug is absorbed at a selected tissue site; and
an ultrasound emitter (3) of an ultrasound apparatus, the ultrasound emitter being coupled in use to an ultrasound source for producing ultrasound energy, the ultrasound emitter (3) being placed on the drug delivery catheter (2) at, or proximal of, the distal end of the catheter (2), wherein in use the ultrasound emitter (3) is configured to direct the ultrasound energy to the selected tissue site to activate the light sensitive drug.

2. The tool of claim 1, wherein at least a portion of the ultrasound emitter (3) is coated with a biocompatible material.

3. The tool of claim 1, wherein the ultrasound emitter (3) is positioned at an exterior of the catheter (2).

4. The tool of claim 1, wherein the ultrasound emitter (3) is positioned at an exterior of the catheter (2) and configured to focus ultrasound energy in a blood vessel lesion.

5. The tool of claim 1, wherein the ultrasound emitter (3) is positioned in a wall of the catheter (2).

6. The tool of claim 1, wherein the ultrasound emitter is positioned in a lumen of the catheter.

7. The tool of claim 1, wherein the catheter (2) is configured to be positioned in an interior of a blood vessel (1).

8. The tool of claim 7, wherein the ultrasound emitter (3) is arranged to deliver the ultrasound energy to a blood vessel lesion in the blood vessel (1).

9. The tool of claim 1, wherein the ultrasound emitter (3) is configured to direct ultrasound energy into a wall of a blood vessel following mechanical angioplasty.

10. The tool of claim 1, wherein the delivery catheter (2) is configured to inject the light sensitive drug into a patient's circulatory system.

11. The tool of claim 1, wherein the delivery catheter (2) is configured to inject the light sensitive drug into a patient's blood vessel.

12. The tool of claim 1, wherein the emitter (3) is configured to direct ultrasound energy in a lateral direction relative to a longitudinal axis of the ultrasound emitter.

13. The tool of claim 1, wherein the emitters (3) configured to direct ultrasound energy in a parallel direction relative to a longitudinal axis of the ultrasound emitter.

14. The tool of claim 1. wherein the emitter (3) is configured to generate 0.02 to 10 W/cm² at a frequency of 20 KHz to 10 MHz.

15. The tool of claim 1, wherein the emitter (3) is configured to generate 0.3W/cm² at a frequency of 1.3 MHz.

16. The tool of claim 1, wherein the ultrasound emitter (3) has a cylindrical, a rectangular or a conical geometry.

17. The tool of claim 1. wherein the ultrasound emitter (3) has a non-circular cross-section geometry.

18. The tool of claim 1, wherein the ultrasound emitter (3) comprises multiple components.

19. The tool of claim 1, wherein the light sensitive drug is one of porphyrin chlorin, acridine derivative, rhodamine, tetracycline, photofrin, UpD, dihematoporphrin and ether, chloroalunum sulfonated phthalocyanine. 8-methoxypsoralen. or 5-amino-levulinic acid.

## Patentansprüche

1. Arzneimittelzuführungsgerät, das aufweist:
einen Arzneimittelzuführungskatheter (2) für das Einführen in einen Patientenkörper, wobei der Arzneimittelzuführungskatheter (2) ein distales Ende hat,
ein lichtempfindliches Arzneimittel, das mittels Ultraschallenergie aktivierbar ist, wobei das lichtempfindliche Arzneimittel über den Arzneimittetzuführungskatheter (2) in den Körper des Patienten einführbar ist, wobei zumindest ein Teil des lichtempfindlichen Arzneimittels an einem ausgewählten Gewebeort absorbiert wird, und
einen Ultraschallsender (3) eines Ultraschallgerätes, wobei der Ultraschallsender im Gebrauch mit einer Ultraschallquelle für das Erzeugen von Ultraschallenergie verbunden ist, wobei der Ultraschallsender (3) auf dem Arzneimittelzuführungskatheter (2) an oder in der Nähe von dem distalen Ende des Katheters (2) plaziert ist, wobei der Ultraschallsender (3) im Gebrauch derart konfiguriert ist, daß er die Ultraschallenergie zu dem ausgewählten Gewebeort richtet, um das lichtempfindliche Arzneimittel zu aktivieren.

2. Gerät nach Anspruch 1, wobei zumindest ein Abschnitt des Ultraschallsenders (3) mit einem biokompatiblen Material beschichtet ist.

3. Gerät nach Anspruch 1, wobei der Ultraschallsender (3) außerhalb des Katheters (2) positioniert ist.

4. Gerät nach Anspruch 1, wobei der Ultraschallsender (3) außerhalb des Katheters (2) positioniert ist und derart konfiguriert ist, daß er Ultraschallenergie in einer Blutgefäßläsion fokussiert.

5. Gerät nach Anspruch 1, wobei der Ultraschallsender (3) in einer Wand des Katheters (2) positioniert ist.

6. Gerät nach Anspruch 1, wobei der Ultraschallsender (3) in einem Lumen des Katheters positioniert ist.

7. Gerät nach Anspruch 1, wobei der Katheter (2) derart konfiguriert ist, daß er in einem Inneren eines Blutgefäßes (1) positioniert wird.

8. Gerät nach Anspruch 7, wobei der Ultraschallsender (3) derart angeordnet ist, daß er die Ultraschallenergie zu einer Blutgefäßläsion in dem Blutgefäß (1) liefert.

9. Gerät nach Anspruch 1, wobei der Ultraschallsender (3) derart konfiguriert ist, daß er Ultraschallenergie in eine Wand eines Blutgefäßes richtet, folgend einer mechanischen Angioplastie.

10. Gerät nach Anspruch 1, wobei der Zuführungskatheter (2) derart konfiguriert ist, daß er das lichtempfindliche Arzneimittel in ein Kreislaufsystem eines Patienten injiziert.

11. Gerät nach Anspruch 1, wobei der Zuführungskatheter (2) derart konfiguriert ist, daß er das lichtempfindliche Arzneimittel in ein Blutgefäß eines Patienten injiziert.

12. Gerät nach Anspruch 1, wobei der Sender (3) derart konfiguriert ist, daß er Ultraschallenergie in einer seitlichen Richtung in bezug auf eine Längsachse des Ultraschallsenders lenkt.

13. Gerät nach Anspruch 1, wobei der Emitter (3) derart konfiguriert ist, daß er Ultraschallenergie in einer parallelen Richtung in bezug auf die Längsachse des Ultraschallsenders richtet.

14. Gerät nach Anspruch 1, wobei der Sender (3) derart konfiguriert ist, daß er 0,02 bis 10 W/cm² mit einer Frequenz von 20 KHz bis 10 MHz erzeugt.

15. Gerät nach Anspruch 1, wobei der Sender (3) derart konfiguriert ist, daß er 0,3 W/cm² mit einer Frequenz von 1,3 MHz erzeugt.

16. Gerät nach Anspruch 1, wobei der Ultraschallsender (3) eine zylindrische, eine rechtekkige oder eine konische Geometrie hat.

17. Gerät nach Anspruch 1, wobei der Ultraschallsender (3) eine nicht kreisförmige Querschnittsgeometrie hat.

18. Gerät nach Anspruch 1, wobei der Ultraschallsender (3) mehrere Komponenten aufweist.

19. Gerät nach Anspruch 1, wobei das lichtempfindliche Arzneimittel Porphyrin, Chlor, Acridinderivativ, Rhodamin, Tetrazyklin, Photofrin, UpD, Dihematoporphrin und Ether, chloroalunumsulfoniertes Phthalocyanin, 8-Methoxypsoralen oder 5-Amino-Levulinicsäure ist.

## Revendications

1. Dispositif pour administrer des substances médicamenteuses, comprenant :
un cathéter de distribution de médicament (2) pour insertion dans le corps d'un patient, le cathéter de distribution de médicament (2) comportant une extrémité distale ;
un médicament photosensible qui peut être activé par une énergie ultrasonore, le médicament photosensible étant adapté pour être introduit dans le corps du patient via le cathéter de distribution de médicament (2), dans lequel au moins une partie du médicament photosensible est absorbée à un site de tissu sélectionné ; et
un émetteur d'ultrasons (3) d'un appareil à ultrasons, l'émetteur d'ultrasons étant couplé, à l'utilisation, à une source d'ultrasons pour produire une énergie ultrasonore, l'émetteur d'ultrasons (3) étant placé sur le cathéter de distribution de médicament (2) à, ou à proximité de, l'extrémité distale du cathéter (2), dans lequel, à l'utilisation, l'émetteur d'ultrasons (3) est configuré pour diriger l'énergie ultrasonore sur le site de tissu sélectionné pour activer le médicament photosensible.

2. Dispositif selon la revendication 1, dans lequel au moins une partie de l'émetteur d'ultrasons (3) est revêtu d'un matériau biocompatible.

3. Dispositif selon la revendication 1, dans lequel l'émetteur d'ultrasons (3) est positionné à l'extérieur du cathéter (2).

4. Dispositif selon la revendication 1, dans lequel l'émetteur d'ultrasons (3) est positionné à l'extérieur du cathéter (2) et configuré pour concentrer l'énergie ultrasonore sur une lésion de vaisseau sanguin.

5. Dispositif selon la revendication 1, dans lequel l'émetteur d'ultrasons (3) est positionné dans une paroi du cathéter (2).

6. Dispositif selon la revendication 1, dans lequel l'émetteur d'ultrasons est positionné dans une lumière du cathéter.

7. Dispositif selon la revendication 1, dans lequel le cathéter (2) est configuré pour être positionné à l'intérieur d'un vaisseau sanguin (1).

8. Dispositif selon la revendication 7, dans lequel l'émetteur d'ultrasons (3) est agencé pour distribuer l'énergie ultrasonore sur une lésion de vaisseau sanguin dans le vaisseau sanguin (1).

9. Dispositif selon la revendication 1, dans lequel l'émetteur d'ultrasons (3) est configuré pour diriger l'énergie ultrasonore dans une paroi d'un vaisseau sanguin après une angioplastie mécanique.

10. Dispositif selon la revendication 1, dans lequel le cathéter de distribution (2) est configuré pour injecter le médicament photosensible dans le système circulatoire d'un patient.

11. Dispositif selon la revendication 1, dans lequel le cathéter de distribution (2) est configuré pour injecter le médicament photosensible dans le vaisseau sanguin d'un patient.

12. Dispositif selon la revendication 1, dans lequel l'émetteur (3) est configuré pour diriger l'énergie ultrasonore dans une direction latérale relativement à un axe longitudinal de l'émetteur d'ultrasons.

13. Dispositif selon la revendication 1, dans lequel l'émetteur (3) est configuré pour diriger l'énergie ultrasonore dans une direction parallèle relativement à un axe longitudinal de l'émetteur d'ultrasons.

14. Dispositif selon la revendication 1, dans lequel l'émetteur (3) est configuré pour produire 0,02 à 10 W/cm² à une fréquence de 20 kHz à 10 MHz.

15. Dispositif selon la revendication 1, dans lequel l'émetteur (3) est configuré pour produire 0,3 W/cm² à une fréquence de 1,3 MHz.

16. Dispositif selon la revendication 1, dans lequel l'émetteur d'ultrasons (3) a une géométrie cylindrique, rectangulaire ou conique.

17. Dispositif selon la revendication 1, dans lequel l'émetteur d'ultrasons (3) a une géométrie en section transversale non circulaire.

18. Dispositif selon la revendication 1, dans lequel l'émetteur d'ultrasons (3) comprend des pièces multiples.

19. Dispositif selon la revendication 1, dans lequel le médicament photosensible est sélectionné dans le groupe consistant en porphyrine, chlorine, dérivé d'acridine, rhodamine, tétracycline, porfimère sodique, UpD, dihématoporphyrine et éther, phthalocyanine sulfonée au chloroalunum, 8-méthoxypsoralène, ou 5-amino-lévulique acide.
